# EUROPÄISCHE PATENTSCHRIFT

(11) **EP 1 362 561 B1**
(45) Veröffentlichungstag und Bekanntmachung des Hinweises auf die Patenterteilung: **03.05.2006**
(21) Anmeldenummer: 03011208.0
(22) Anmeldetag: 16.05.2003
(51) Int. Cl.: A61C 8/00, A61B 17/58

(54) **Vorrichtung zum ablösen einer Kieferhöhlenschleimhaut, die eine Perforation feststellt**
Mandibular sinus membrane separator capable of detecting tissue perforation
Dispositif pour séparer la membrane d'un sinus mandibulaire, avec indicateur de perforation

(30) Priorität: 17.05.2002 DE 10222636
(43) Veröffentlichungstag der Anmeldung: 19.11.2003
(73) Patentinhaber: Mahmoudi-Lamouki, Mohammad, 45133 Essen (DE); Balan, Roland, 73732 Esslingen (DE)
(72) Erfinder: Mahmoudi-Lamouki, Mohammad, 45133 Essen (DE); Balan, Roland, 73732 Esslingen (DE)
(74) Vertreter: Rüger, Barthelt & Abel Patentanwälte

(56) Entgegenhaltungen:
- EP-A- 1 174 094
- WO-A-00/49978
- WO-A-01/85062
- US-A- 5 711 315
- US-A- 5 792 100

## Beschreibung

Das Knochenangebot ist im Oberkiefer von Natur aus durch die Struktur der Kieferhöhle (Sinus) in seiner Höhe begrenzt. Wenn sich zudem durch Zahnverlust und/oder Tragen einer schlecht sitzenden Prothese der Kiefer im Seitenzahnbereich stark zurückgebildet hat, besteht häufig zu wenig Knochenhöhe für Implantate. Abhilfe kann durch eine Sinusbodenelevation oder -augmentation, also durch eine Anhebung des Kieferhöhlenbodens geschaffen werden. Bei diesem Eingriff wird im Oberkieferseitenzahnbereich vom Mundvorhof aus Knochenersatzmaterial oder Eigenknochen eingebracht, der nach seiner Einheilung als neues Implantatlager dient.

Bei aus der Praxis bekannten Verfahren wird vor der Einbringung des Knochenersatzmaterials die Kieferhöhle geeignet präpariert. Hierzu wird der Kieferknochen an einer Stelle durchtrennt und falltürartig in die Kieferhöhle eingeklappt, wobei er sich gegen die die Kieferhöhle auskleidende Schleimhaut abstützt und diese vom Sinusboden abhebt. Anschließend wird die Kieferhöhlenschleimhaut durch den so geschaffenen Zugang mit Handinstrumenten, teils unter Zuhilfenahme von Endoskopen, sukzessiv von dem Kieferknochen abgehoben. Wenn der entsprechende Sinusbodenbereich freigelegt ist, kann die Sinusbodenelevation durchgeführt werden.

Eine der größten Schwierigkeiten bei der Präparation der Kieferhöhle besteht darin, die Kieferhöhlenschleimhaut nicht zu verletzen. Die Kieferhöhlenschleimhaut ist bei verschiedenen Individuen unterschiedlich dick und dementsprechend schwerer oder leichter zu präparieren. Die knöchernen Septen der Kieferhöhle und deren Kanten stellen Prädilektionsstellen für Risse dar, so dass trotz Erfahrungen des Operateurs im Umgang mit den Instrumenten Einrisse der Schleimhaut nicht zu vermeiden sind. Die Integrität dieser Membran ist aber für den Erfolg der Sinusbodenelevation von großer Bedeutung. Deren Perforation kann dazu führen, dass das Augmentationsmaterial von der Kieferhöhlenseite einer Kontamination zugänglich wird oder über die Nase abwandert. Die Folgen der Verletzung der Kieferhöhlenschleimhaut reichen also von Totalverlust des Augmentationsmaterials, insuffizienter Elevation, die eine Implantation nicht mehr rechtfertigt, bis hin zu chronischen, zuweilen stummen Infekten, die eventuell Explantationen nach sich ziehen.

Bislang wurden Einrisse durch resorbierbare Membranen unterhalb der Kieferhöhlenschleimhaut unterfüttert. Die Resorption dieser Membranen erhöht die Sicherheit nur wenig, weil sie zu einer bakteriellen Entzündung von der Kieferhöhlenseite her neigt. Das bis zu 14 Wochen dauernde Zeitfenster der Resorption erhöht die Gefahr einer Sekundärinfektion des darunter liegenden Transplantates. Problematisch ist auch, dass viele Einrisse der Kieferhöhlenschleimhaut überhaupt nicht festgestellt werden.

Das Dokument EP 1174094 offenbart eine vorrichtung zum lösen zweier gewebeschichten voreinander mit einer Fluideinrichtung.

Es ist daher Aufgabe der vorliegenden Erfindung, eine Vorrichtung zu schaffen, die ein Lösen zweier Gewebeschichten voneinander, insbesondere einer Kieferhöhlenschleimhaut von einem Kieferknochen, bei geringem Risiko einer Perforation der zu lösenden Gewebeschicht ermöglicht.

Weiterhin ist es Aufgabe der vorliegenden Erfindung, beim Einsatz der Vorrichtung eine einsetzende Verletzung der zu lösenden Gewebeschicht möglichst früh zu erkennen, um das Trauma auf ein Minimum zu reduzieren.

Diese Aufgaben werden durch die Vorrichtung mit den Merkmalen des Anspruchs 1 gelöst.

Bei der erfindungsgemäßen Vorrichtung wird also zu Präparations- und/oder Diagnosezwecken ein gesteuerter oder geregelter Fluidstrom verwendet. Das Fluid wird von der Pumpvorrichtung mit definiert zu steuerndem oder regelndem Volumen über die Fluidleitung bis zu einem Zugang zu den Gewebeschichten gefördert. Der Fluidstrahl wird derart in den Hohlraum zwischen den Gewebeschichten eingeleitet, dass die zu lösende Gewebeschicht von der anderen, als Unterlage dienenden Gewebeschicht schonend angehoben wird. Im Vergleich zum Einsatz konventioneller Handinstrumente ist die Gefahr einer Verletzung der zu lösenden Gewebeschicht dadurch reduziert.

Die Sensoreinrichtung dient dazu, beim Einsatz der Vorrichtung den Druck im Strömungsweg des Fluids zu erfassen und den Druckwerten entsprechende Signale für die Steuerungseinrichtung zu generieren. Die Steuerungseinrichtung wertet diese Signale aus, um das zu pumpende Volumen zu steuern oder zu regeln und um auftretende unzulässige Druckveränderungen festzustellen. Es können bereits geringe Druckverluste, die auf eine Penetration oder mikrofeine Verletzungen der zu lösenden Gewebeschicht schließen lassen, wie auch signifikante Druckverluste, die eine Perforation der Gewebeschicht kennzeichnen, bereits im frühen Stadium festgestellt werden. Durch geeignete Maßnahmen, z.B. manuelle oder automatische Reduzierung des Fluidstroms oder Abschaltung der Pumpvorrichtung, kann rechtzeitig eine weitere Verletzung der Membran verhindert werden. Außerdem wird die erforderliche diagnostische Sicherheit während der Präparation der Gewebeschichten sichergestellt. Die erfindungsgemäße Vorrichtung kann nur als Diagnosegerät oder als Präparationsgerät mit gleichzeitiger Diagnosemöglichkeit verwendet werden.

Die erfindungsgemäße Vorrichtung ist insbesondere zur Anwendung bei der Präparation der Kiefernhöhle geeignet, um eine Sinusbodenelevation vorzubereiten bzw. durchzuführen. Die als Unterlage dienende erste Gewebeschicht ist dann also ein Kieferhöhlenknochen und die Kieferhöhlenschleimhaut (Schneidersche Membran) stellt die anzuhebende zweite Gewebeschicht dar. Durch die Vorteile der erfindungsgemäßen Vorrichtung kann das Trauma bei der Präparation auf ein Minimum reduziert werden. Jedenfalls wird dadurch die Erfolgswahrscheinlichkeit der Sinusbodenelevation a priori wesentlich erhöht. Außerdem kann der Zugang, bspw. ein in den Kieferknochen auszuführendes rundes oder falltürartiges Trepanationsloch, relativ klein ausgeführt werden. Es sind noch andere Anwendungsfälle bei denen eine Gewebeschicht oder Membran von einer steifen oder flexiblen Unterlage, bspw. einem Muskel oder Knochen zu lösen ist, z.B. für die Schädelhöhle oder Augenhöhle, möglich.

Das Fluid ist je nach Bedarf ein Gas oder ein Gasgemisch, insbesondere sterilisierte Luft, eine Flüssigkeit, insbesondere physiologische Kochsalzlösung, oder ein Gas-Flüssigkeitsgemisch. Es können oberflächenaktive, nichttoxische Substanzen, sogenannte Surfactants, beigefügt werden, um die Ablösewirkung zu unterstützen und auch sehr dünne fragile Membranen sicher zu präparieren. Gegebenenfalls kann für eine Sinusbodenelevation sofort flüssiges Knochenersatzmaterial, sofern erhältlich, durch das Trepanationsloch eingespritzt werden. Für bildgebende Verfahren können Kontrastmittel, wie Röntgenkontrastmittel oder dgl., eingebracht werden.

Die Pumpvorrichtung ist vorzugsweise eine Dosierpumpe, die pro Umlauf ein definiertes Volumen an Fluid in die Fluidleitung pumpt. Statt einer aktiven Pumpe kann zu Zwecken der Präparation der Kieferhöhlenschleimhaut an dem proximalen Ende der Fluidleitung auch lediglich ein Ventil verwendet werden. Das Ventil öffnet in den Phasen des Einatmens und führt unter die Kieferhöhlenschleimhaut bspw. Luft zu. In den Phasen des Ausatmens schließt das Ventil, so dass das erzeugte Volumen in dem Raum zwischen der Kieferhöhlenschleimhaut und dem Kieferknochen verbleibt.

Die Fluidleitung ist vorzugsweise durch einen flexiblen Schlauch gebildet, der im einfachsten Fall in die Zugangsöffnung, insbesondere das Trepanationsloch, eingeführt und darin von Hand gehalten wird. Es ist aber vorzugsweise ein geeignetes Befestigungsmittel vorgesehen, mit dem die Fluidleitung oder der Schlauch in unmittelbarer Nachbarschaft der Zugangsöffnung angebracht und gehalten werden kann, um eine nach außen hin dichte und sichere Strömungsverbindung mit der Zugangsöffnung zu erhalten.

Vorteilhafterweise ist das Befestigungsmittel in Form eines an einem Ende der Fluidleitung angeordneten Saugnapfes ausgebildet, der eine einfache und dichte Anbringung der Fluidleitung ermöglicht.

Bei einer besonders vorteilhaften Ausführungsform ist ein Doppelsaugnapf vorgesehen. Dieser besteht aus zwei unterschiedlich großen, zueinander konzentrischen Saugnäpfen, wobei der größere Saugnapf längs der Fluidleitung in Richtung auf den kleineren Saugnapf zu und von diesem weg beweglich angeordnet ist. Nach Anbringung beider Saugnäpfe wird eine besonders gute Abdichtung und Haftung erhalten, wobei die Haftkraft durch Einführen eines Gels zwischen den beiden Saugnäpfen weiter erhöht werden kann.

Bei einer Zugangsöffnung von 2 bis 3 mm, wie sie bei der Präparation der Kieferhöhlenschleimhaut vorzugsweise vorzusehen ist, kann der erste Saugnapf einen äußeren Durchmesser von etwa 3 bis 4 mm aufweisen. Der entsprechende Durchmesser bei dem zweiten Saugnapf beträgt dann ca. 7 bis 16 mm, vorzugsweise 7 bis 9 mm, je nach dem zu verwendenden Fluid.

Zu der erfindungsgemäßen Sensoreinrichtung gehört wenigstens ein im Strömungsweg angeordneter Drucksensor. Im einfachsten Fall ist dieser Sensor direkt am Pumpenausgang angeordnet. Reißt die zu lösende Gewebeschicht ein, so wird dies von der Steuerungseinrichtung in Form eines Druckverlustes registriert.

Bei einer anderen Ausführungsform ist der oder ein zusätzlicher Sensor am Ende der Fluidleitung, also unmittelbar an dem Zugang zu den Gewebeschichten, bspw. dem Trepanationsloch angeordnet. Druckabfälle und Druckverluste in der Fluidleitung spielen dann kaum eine Rolle. Die Präparation der Gewebeschichten kann viel genauer überwacht werden. Der Druck kieferhöhlenseitig unter der zu präparierenden Gewebeschicht in Abhängigkeit von dem präparierten Volumen liefert eine geeignete Regelgröße. Reaktionszeiten sind kürzer.

Die Steuereinrichtung überwacht während des Einsatzes der erfindungsgemäßen Vorrichtung die Druckverhältnisse, indem sie den momentan erfassten Istwert des Drucks des Volumenstroms mit einem Sollwert oder einem Sollbereich vergleicht. Der Sollbereich kann durch einen oder mehrere Schwellenwerte definiert sein, der oder die von der Bedienperson mittels einer Eingabeeinrichtung vorgegeben oder von der Steuereinrichtung automatisch bestimmt wird bzw. werden. Bei der Festlegung des Sollbereichs können Größen wie Art und Dichte des Fluids mit berücksichtigt werden. Gegebenenfalls werden auch Länge und Durchmesser der Fluidleitung und/oder objektspezifische Faktoren, wie bspw. der Typ der zu präparierenden Kieferhöhlenschleimhaut, mit berücksichtigt. Der Schwellenwert oder Sollbereich kann während des Ablösevorgangs im Hinblick auf die veränderten Verhältnisse geeignet angepasst werden.

Die Pumpe kann auch von einem Motor angetrieben sein. In diesem Fall ist die Motorleistung für den Druck im Strömungsweg kennzeichnend und wird vorteilhafterweise von der Sensoreinrichtung überwacht. Bei variablem Volumenstrom kann die Sensoreinrichtung zusätzlich auch diesen erfassen und auswerten.

Es ist vorzugsweise eine optische und/oder akustische Anzeigeeinrichtung vorgesehen, um beim Feststellen eines unzulässigen Unterschreiten und gegebenenfalls Überschreiten des Drucksollbereichs ein für die Bedienperson wahrnehmbares Warnsignal zu generieren.

Die Steuereinrichtung steuert oder regelt die Pumpvorrichtung, so dass diese den gewünschten Volumenstrom pumpt. Der Volumenstrom wird vorzugsweise in Abhängigkeit von den bereits erwähnten, für das Fluid, den Strömungsweg und/oder das Objekt kennzeichnenden Faktoren geregelt.

Bei der Präparation der Kieferhöhlenschleimhaut wird bei der Steuerung bzw. Regelung des Volumenstroms vorzugsweise auch die Atmung des Patienten, insbesondere die mittlere Länge der Phasen des Ein- und Ausatmens und die dadurch verursachte Druckdifferenz in der Kieferhöhle, mit berücksichtigt. Das Fluid kann vorteilhafterweise nur während des Einatmens gepumpt werden, während die Phasen des Ausatmens zur natürlichen Verteilung des Präparationsmittels unter der Kieferhöhlenschleimhaut genutzt werden. Dabei kann die erfindungsgemäße Vorrichtung die Erfassung der Phasen des Ein- und Ausatmens in regelmäßiger Folge vornehmen, um sich fortwährend neu derart zu kalibrieren, dass eine wirksame Präparation ohne Einriss der Kieferhöhlenschleimhaut möglich ist.

Weitere vorteilhafte Einzelheiten von Ausführungsformen der Erfindung ergeben sich aus Unteransprüchen, der Zeichnung sowie der zugehörigen Beschreibung. In der Zeichnung ist ein Ausführungsbeispiel des Gegenstandes der Erfindung dargestellt. Es zeigen:
- Fig. 1: einen Schnitt durch einen Kieferhöhlenknochen mit daran angeschlossener Vorrichtung zur Präparation der Kieferhöhlenschleimhaut, in einer stark schematisierten Darstellung,
- Fig. 2: einen Ausschnitt der in Fig. 1 gezeigten Kieferhöhle während der Präparation der Kieferhöhlenschleimhaut mit einem bei der erfindungsgemäßen Vorrichtung nach Fig. 1 verwendeten Befestigungs- und Dichtmittel, in einer vergrößerten Darstellung, im Querschnitt, und
- Fig. 3: eine weitere Ausführungsform einer erfindungsgemäßen Vorrichtung zum Lösen zweier Gewebeschichten voneinander, in einer stark schematisierten Darstellung.

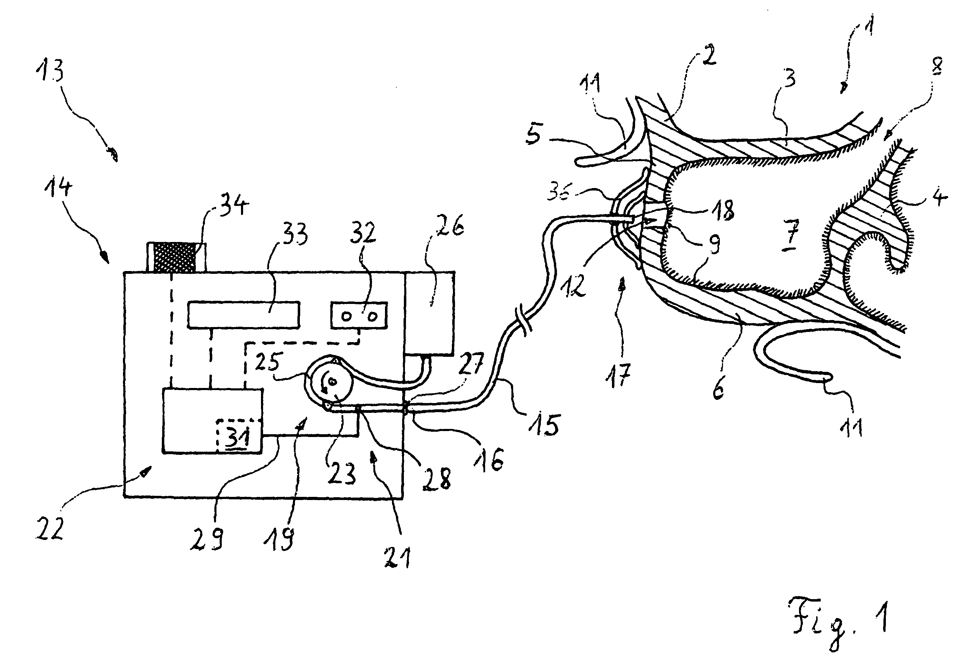

In Fig. 1 ist stark schematisiert ein Schnitt durch einen Seitenzahnbereich eines menschlichen Oberkiefers 1 dargestellt. Es ist ein Kieferknochen 2 veranschaulicht, der hier im Wesentlichen vier Wandabschnitte 3, 4, 5 und 6 aufweist, die eine Kieferhöhle 7, sogenannten "Sinus", begrenzen. Der in Fig. 1 im Wesentlichen horizontal verlaufende Wandabschnitt 3 legt mit einem lateralen Wandabschnitt 4 eine Mündung 8 fest, die die Kieferhöhle 7 mit einer hier nicht gezeigten Nasenhöhle verbindet. Den Wandabschnitten 3 und 4 gegenüber sind ein lateraler Wandabschnitt 5 und ein in Fig. 1 im Wesentlich horizontal ausgerichteter Wandabschnitt 6 angeordnet, der den Kieferhöhlenboden bildet. Die Wandabschnitte 5 und 6 grenzen an einen Mundvorhof, einen Teil der Mundhöhle an.

An dem Kieferknochen 2 liegt eine Kieferhöhlenschleimhaut 9, auch "Schneidersche Membran" genannt, an und dichtet diesen gegen die Kieferhöhle 7 ab. Auf der Seite zum Mundvorhof hin ist der Kieferknochen 2 von einem Mucoperiostlappen 11, bedeckt, die hier bereits durchtrennt und zum Teil von dem Kieferknochen 2 abgezogen gezeigt ist; so dass ein freier Zugang zu dem Kieferknochen 2 geschaffen ist. In dem freigelegten Bereich des lateralen Wandabschnitts 5 ist eine Zugangsöffnung 12 ausgebildet, die durch den Kieferknochen 2 hindurch bis zu der Kieferhöhlenschleimhaut 9 führt.

Weiterhin ist in Fig. 1 eine erfindungsgemäße Vorrichtung 13 veranschaulicht, zu der im Wesentlichen ein Steuergerät 14, ein flexibler Schlauch 15, der mit seinem einen Ende 16 an das Steuergerät 14 angeschlossen ist, sowie ein Befestigungsmittel 17 gehören, das das andere, freie Ende 18 des Schlauches 15 an dem Kieferknochen 2 fixiert.

Das Steuergerät 14 umfasst eine Pumpe 19, eine Sensoreinrichtung 21 und eine Steuerungseinrichtung 22, die hier lediglich schematisch dargestellt sind. Die Pumpe 19 ist eine Dosierpumpe, die eine im Betrieb umlaufende Scheibe 23 aufweist, an deren Umfang mehrere, im vorliegenden Fall zwei Kugellager vorgesehen sind. Mit Hilfe dieser Kugellager verdrängt die Pumpe 19 pro halbe Umdrehung ein definiertes Volumen eines Fluids aus einer um den Umfang der Scheibe 23 herum angeordneten Leitung 25. Ein Ende der Leitung 25 ist mit einer Fluidquelle 26 strömungsmäßig verbunden und das andere Ende der Leitung 25 führt zu einem Fluidausgang 27 des Steuergeräts 14. Es sind auch andere Pumpenarten, wie Zahnradpumpen oder Zylinder-Kolben-Einheiten, die pro Hub ein definiertes Volumen hinaus pumpen, verwendbar.

Die Sensoreinrichtung 21 weist einen in der Nähe des Pumpenausgangs bzw. Fluidausgang 27 in oder an der Leitung 25 angeordneten Drucksensor 28 auf, der unmittelbar den dortigen Druck als Messgröße aufnimmt und zur Weiterverarbeitung in ein geeignetes elektrisches Signal umwandelt. Solche Drucksensoren sind aus der Technik allgemein bekannt und brauchen hier nicht näher erläutert zu werden. Auf Widerstandmessung basierende Dehnungsmessstreifen oder piezoresisistive Sensoren werden vorgezogen. Es sind aber auch induktive, kapazitive oder piezoelektrische Sensoren verwendbar oder auch Manometer, die ein mechanisches Signal abgeben, einsetzbar.

Der Drucksensor 28 ist über eine elektrische Leitung 29 mit der Steuereinrichtung 22 verbunden, deren Aufgabe es ist, die über die Leitung 29 empfangenen Signale, die den Druck in der Leitung 25 kennzeichnen, auszuwerten. Die Steuereinrichtung 22 weist hierzu eine hier durch das Bezugszeichen 31 angedeutete Verarbeitungseinheit auf, bspw. einen Prozessor, der den momentan erfassten Druckwert auf Zulässigkeit überprüft. Ist der Wert unzulässig, z.B. zu hoch oder zu niedrig, bewirkt die Steuereinrichtung 22 eine Reduktion der Pumpenleistung oder schaltet die Pumpe 19 ab.

Das Steuergerät 14 weist außerdem eine Bedieneinheit 32 auf, über die der Volumenstrom, z.B. in Form der Drehzahl der Pumpe 19, und der Sollbereich für Druckwerte in der Leitung 25 oder entsprechende Schwellenwerte vorgegeben werden können. Die eingegebenen Werte werden von der Verarbeitungseinheit 31 abgespeichert und zur Steuerung der Pumpe 19 und zur Überwachung des Drucks herangezogen.

Weiterhin enthält das Steuergerät 14 wahlweise eine mit der Steuereinrichtung 22 gekoppelte Anzeige 33, auf der die momentan eingestellten und gemessenen Werte wie Druck Volumenstrom, der vorzugsweise auch erfasst wird, angezeigt werden können. Bei einem Störfall, bspw. einer abrupten Minderung des gemessenen Druckwerts, wird zudem ein akustisches Signal über einen an dem Steuergerät 14 vorgesehenen Lautsprecher 34 ausgegeben.

Der als Fluidleitung dienende flexible Schlauch 15 ist mit seinem einen Ende 16 an den Fluidausgang 27 des Steuergeräts 14 angeschlossen. Sein anderes Ende 18 ist mittels des Befestigungsmittels 17 an dem lateralen Wandabschnitt 5 des Kieferknochens 2 angebracht und steht mit der Zugangsöffnung 12 in Strömungsverbindung.

Das Befestigungsmittel 17 ist in der Fig. 2 stark vergrößert dargestellt. Wie ersichtlich gehören zu dem Befestigungsmittel 17 zwei glockenförmige Saugnäpfe 35 und 36, die an dem Ende 18 des Schlauchs 15 auf diesen aufgeschoben sind. Die Saugnäpfe 35 und 36 sind aus einem elastischen Material, vorzugsweise als auswechselbare Einmalartikel gefertigt. Eine erster Saugnapf 35 ist unmittelbar an dem Ende 18 des Schlauchs 15 angeordnet und stützt sich mit seinem ringförmigen äußeren Rand 37 gegen den Wandabschnitt 5 ab. Der Rand 37 umgibt die Zugangsöffnung 12, und das Ende 18 des Schlauchs 15 befindet sich in unmittelbarer Nähe zu der Zugangsöffnung 12.

Der zweite Saugnapf 36 ist auf dem Schlauch 15 von dem Saugnapf 35 axial beabstandet angeordnet und weist einen äußeren Rand 38 mit einem größeren Durchmesser auf als der Durchmesser des Randes 37 des Saugnapfs 35. Der Saugnapf 36 sitzt mit seinem nabenartigen inneren Rand 39 verschiebbar auf dem Schlauch 15, so dass er in Richtung auf den Saugnapf 35 zu und von diesem weg bewegt werden kann. Nach Befestigung umgibt der äußere Saugnapf 36 den inneren Saugnapf 35. Die Verbindung zwischen der Nabe 39 und dem Schlauch 15 ist trotz Verschiebbarkeit nach außen dicht. Durch Andrücken des Saugnapfs 36 gegen den lateralen Kieferknochenabschnitt 5 wird in dem Raum zwischen den beiden Saugnäpfen 35 und 36 ein Unterdruck erzeugt, der ausreicht, um das Befestigungsmittel 17 mit dem Schlauch 15 sicher an dem Kieferknochenabschnitt 5 zu halten. Zur Erhöhung der Haftwirkung ist in dem Zwischenraum zwischen den beiden Saugnäpfen ein kompressibles Gel 40 eingebracht. Zum Erleichtern des Erzeugens des Unterdrucks und des Lösens des Befestigungsmittels 17 ist an dem Saugnapf 36 eine mit einem Stopfen oder dgl. verschießbare Entlüftungsöfnung 41 vorgesehen. Zur Sicherheit kann der Schlauch 15 auch von Hand in der Zugangsöffnung 12 gehalten werden, wobei der Doppelsaugnapf 35, 36 eine besondere Abdichtung der Öffnung 12 ermöglicht. Es kann auch eine dreifache Dichtung vorgesehen werden.

Die insoweit beschriebene erfindungsgemäße Vorrichtung 13 wird vorzugsweise zur Präparation der Kieferhöhlenschleimhaut 9 verwendet. Sie funktioniert wie folgt:

Es wird angenommen, dass die Vorrichtung bereits funktionsfähig ist, insbesondere die Leitung 25 mit der Fluidquelle 26 verbunden und der Schlauch 15 an den Fluidausgang 27 des Steuergeräts angeschlossen ist. Weiterhin wird angenommen, dass das Operationsgebiet, nämlich die Wandabschnitte 5 und 6, wie oben beschrieben, durch Bildung des Mucoperiostlappens 11 vorbereitet wurde. Dann wird vom Mundvorhof aus mit einem chirurgischen Bohrer, einem Rosenbohrer oder einer Diamantkugel, zunächst das initiale Trepanationsloch 12 in dem lateralen Kieferknochenabschnitt 5 hergestellt, um einen Zugang zu der Kieferhöhlenschleimhaut 9 zu erhalten. Dabei wird dem rotierenden Bohrerkopf zur Kühlung Luft, Wasser oder physiologische Kochsalzlösung zugeführt. Durch den dabei entstehenden Spraynebel wird die Kieferhöhlenschleimhaut 9 kieferhöhlenseitig ausgelenkt und ferngehalten und somit geschont, wie dies aus herkömmlichen Verfahren bekannt ist.

Anschließend wird die Vorrichtung 13 mittels des Schlauches 15 an das Trepanationsloch 12 angeschlossen, das durch Andrücken des Doppelsaugnapfes 35, 36 nach außen abgedichtet wird. Mittels der Bedieneinheit 32 werden eine zweckmäßige Größe für den zu pumpenden Volumenstrom sowie eine zulässige Schwankungsbreite für den Druck in der Leitung 25 vorgegeben. Hierbei werden vorzugsweise die Art und die Dichte des Fluids sowie möglichst auch die Länge und der Durchmesser der Fluidleitung 15, also der Strömungswiderstand oder die Druckverluste, mit berücksichtigt. Es kann auch der abschätzbare Schleimhauttyp, wie hyper, normal oder astrophisch, eingegeben werden.

Die Pumpe 19 pumpt dann den definierten Volumenstrom des Fluids durch den Schlauch 15 und das Trepanationsloch 12 in den Raum zwischen dem lateralen Wandabschnitt 5 des Kieferknochens 2 und der Kieferschleimhaut 9. Durch den sanften Strahl des Fluids, bspw. Luft, Flüssigkeit oder eines Gas-Flüssigkeitsgemisches, auf die Kieferschleimhaut 9 löst sich diese fortschreitend von den Kieferknochenabschnitten 5 und 6 ab ohne einzureißen. Der Beginn des Anhebens der Kieferschleimhaut 9 ist in der Fig. 2 verdeutlicht.

Bei der Präparation der Kieferschleimhaut 9 nimmt die Sensoreinrichtung 21 den Druckwert in dem Schlauch 25 auf, und die Verarbeitungseinheit 31 der Steuereinrichtung 22 überwacht durch Vergleich des Istwerts des Drucks mit den abgespeicherten Schwellenwerten, dass keine unzulässige Minderung oder Erhöhung des Drucks auftritt.

Stellt die Steuereinrichtung 22 einen unzulässigen Druckanstieg fest, so deutet dies meist auf ein Präparationshindernis, z.B. eine Septe hin. Bei Bedarf kann der Volumenstrom dann reduziert werden, um eine Perforation der Kieferhöhlenschleimhaut 9 zu vermeiden.

Stellt die Steuereinrichtung 22 jedoch einen Druckverlust fest, so ist dies zumindest mit einer Penetration oder bei hohen Druckverlusten sogar mit einer Perforation der Kieferhöhlenschleimhaut 8 gleichzusetzen. Im ersten Fall wird die Bedienperson zumindest über die Anzeige 33 oder den Lautsprecher 34 darauf aufmerksam gemacht. Im letzeren Fall wird die Pumpe 19 vorzugsweise sofort abgeschaltet, um einen größeren Schaden zu vermeiden. Die Bedienperson muss dann entscheiden, ob der Einriss der Kieferhöhlenschleimhaut 9 bspw. mit Hilfe einer resorbierenden Membran unterfüttert werden muss und ob die Präparation der Kieferhöhle 7 fortgesetzt werden kann. Mit der erfindungsgemäßen Vorrichtung lässt sich die Gefahr einer Perforation der Kieferhöhlenschleimhaut 9 bei deren Präparation deutlich reduzieren. Tritt aber eine Perforation auf, so wird dies sicher und rechtzeitig erkannt.

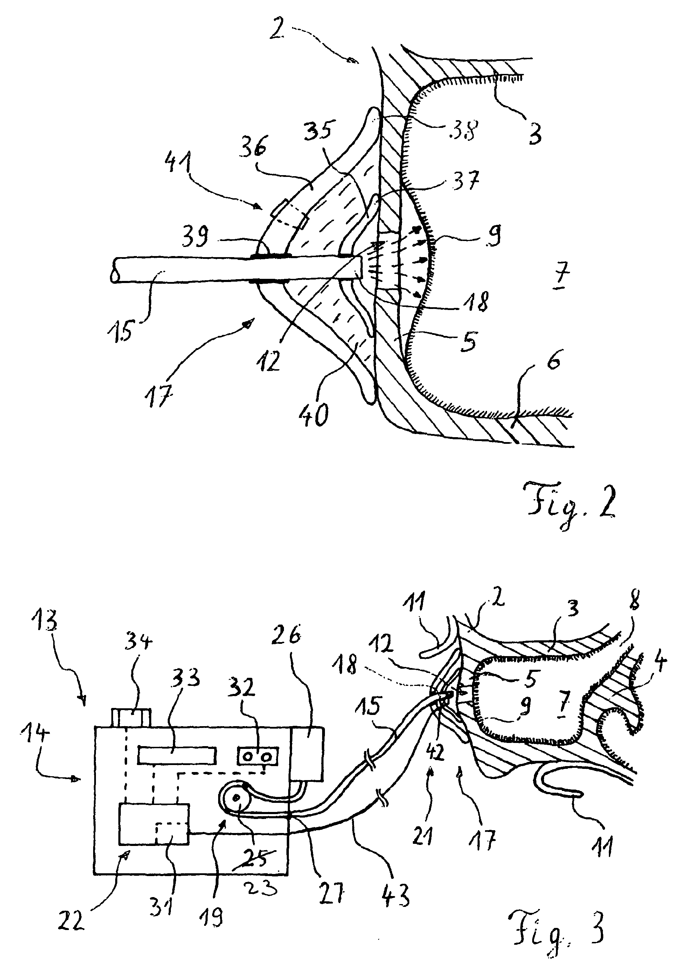

In Fig. 3 ist eine weitere Ausführungsform der erfindungsgemäßen Präparationsvorrichtung 13 veranschaulicht. Soweit Übereinstimmungen mit der vorstehend beschriebenen Vorrichtung in Bau und/oder Funktion bestehen, wird unter Zugrundelegung gleicher Bezugszeichen auf die vorstehende Beschreibung verwiesen.

Im Unterschied zu dem Drucksensor 28 bei der Vorrichtung 12 nach Fig. 1 und 2 ist der Drucksensor 42 der Sensoreinrichtung 21 hier nicht in der Leitung 25, sondern in unmittelbarer Nähe des Trepanationsloches 12 an dem Ende 18 des Schlauches 15 innerhalb des inneren Saugnapfs 35 angeordnet und über eine Signalleitung 43 mit der Steuereinrichtung 22 gekoppelt. Dadurch können auftretende Druckveränderungen viel genauer und reaktionsarmer erfasst und die Gefahr eines Einrisses der Kieferhöhlenschleimhaut 9 noch mehr vermindert werden. Der Drucksensor 42 ist vorzugsweise auswechselbar in dem Saugnapf 35 gehalten, um sterilisiert werden zu können.

Außerdem schafft eine derartige Anordnung des Drucksensors 42 erweiterte Möglichkeiten der Anwendung und Funktionsweise der erfindungsgemäßen Vorrichtung 13. Bei der in Fig. 3 veranschaulichten Ausführungsform werden z.B. die Phasen des Ein- und Ausatmens, also deren Dauer und die dadurch bedingten natürlichen Druckschwankungen in der Kieferhöhle gemessen und automatisch von der Steuereinrichtung 22 bei der Überwachung des Istdrucks einbezogen. Die Steuereinrichtung steuert bzw. regelt die Pumpe 19 derart, dass zyklisch und alternierend einmal das Fluid gefördert und dann die Verhältnisse beim Ein- und Ausatmen erfasst werden. Die Steuereinrichtung passt das zu pumpende Volumen insbesondere durch die Umlaufzahl der Dosierpumpe 19 selbständig an sich verändernde natürliche Druckschwankungen an. Diese Druckschwankungen dienen somit als Regelgröße bei der Regelung des einzubringenden Volumens. Die Vorrichtung 13 rekalibriert sich sozusagen wiederholt, bspw. nach jeweils 0,5 cm³ eingebrachtem Volumen des Fluids, von selbst. Wiederholte Rekalibrierungen können aber Anzeichen für Mikroläsionen der Kieferhöhlenschleimhaut 9 sein und werden ausgewertet. In diesem Fall wird, um weitere Beschädigung zu vermeiden, die Präparation beendet. Treten während der Kalibrierzeit keine natürlichen Druckschwankungen auf, so deutet dies auf eine ausbleibende Ventilation der Nasennebenhöhle hin.

Das zur Präparation verwendete Fluid wird vorzugsweise nur in Phasen des Einatmens eingepumpt (aktive Präparation), in denen auf natürliche Weise ein Unterdruck auf der Kieferhöhlenseite entsteht. Die nachfolgenden Phasen des Ausatmens werden dann zur natürlichen Druckverteilung des Fluids unter die Kieferhöhlenschleimhaut 9 genutzt (passive Präparation). Plötzlichen Druckabfall wird aber in beiden Phasen überwacht. Bei einer Intubationsnarkose ohne Ventilation der Nasennebenhöhlen braucht keine Rücksicht auf den Atemzyklus genommen zu werden.

Mit beiden Ausführungsformen der erfindungsgemäßen Vorrichtung 13 nach Fig. 1 und nach Fig. 3 kann nach der Präparation der Kieferhöhlenschleimhaut 9, also nach deren Lösen und Anheben von dem Kieferknochen 2 in dem zu behandelnden Bereich, weiter wie folgt vorgegangen werden: Es kann ein Absorptionsmittel, im einfachsten Falle physiologische Kochsalzlösung, zu röntgenologischen Untersuchung eingebracht werden. Es kann auch mit Hilfe der Vorrichtung 13 ein flüssiges Augmentationsmaterial direkt über die Zugangsöffnung 1.2 dem Kieferhöhlenboden 6 zugeführt werden, um unter Verdrängung des zuvor zur Präparation verwendeten Fluids den Kieferhöhlenboden 6 vertikal aufzufüllen. Damit kann auf einfache Weise eine Sinusbodenaugmentation sofort nach Präparation der Kieferhöhlenschleimhaut 9 durchgeführt werden. Ansonsten kann der laterale Zugang 12 unter Abhalten der Kieferhöhlenschleimhaut 9 mit einem Kugelstopfer oder dgl. auch erweitert und das Implantatlager durch Einbringen eines geeigneten Knochenersatzmaterials aufbereitet werden. Eine Implantation kann gleichzeitig mit der Sinusbodenelevation oder erst später, nach Einheilung des eingesetzten Knochenmaterials durchgeführt werden. Nach erfolgtem Eingriff wird die durch das Befestigungsmittel 17 gebildete Dichtung gelöst.

Eine Präparationsvorrichtung zur Präparation einer Kiefernhöhle 7 für eine Sinusbodenelevation weist eine gesteuerte oder geregelte Pumpe 19 auf, die ein Präparationsmittel mit definiertem Volumenstrom aus einer entsprechenden Quelle pumpt, eine Schlauchverbindung 15, durch die das Präparationsmittel bis zu einer in dem Kieferknochen 2 auszuführenden Zugangsöffnung 12 gefördert wird, und ein Befestigungsmittel 17 auf, das durch Erzeugung einer Unterdrucks eine Befestigung des Schlauchs 15 an dem Kieferknochen 2 ermöglicht. Der durch die Zugangsöffnung 12 hindurchtretende Strahl des Präparationsmittels wird gegen die Kieferhöhlenschleimhaut 9 gerichtet, um diese sanft von dem Kieferknochen abzulösen, anzuheben und gehoben zu halten.

Eine Sensoreinrichtung 21 dient dazu, den Druckwert im Strömungsweg des Präparationsmittels oder die Motorleistung eines die Pumpe 19 antreibenden Motors zu erfassen. Die erfassten Werten werden von einer Steuerungseinrichtung 22 ausgewertet, um unzulässige Veränderungen der Werte, die auf eine Perforation der Kieferhöhlenschleimhaut 9 schlie-ßen lassen, zu erkennen.

## Patentansprüche

1. Vorrichtung (13) zum Lösen zweier Gewebeschichten (2,9) voneinander und/oder zur Feststellung einer Perforation einer an einer ersten Gewebeschicht (2) haftenden zweiten Gewebeschicht (9)
mit einer Pumpvorrichtung (19), die mit einer Fluidquelle (26) verbindbar und zum Fördern eines Fluids mit zu steuernden oder zu regelendem Volumen eingerichtet ist,
mit einer Fluidleitung (15), deren erstes Ende (16) mit der Fluidquelle (26) strömungsmäßig verbindbar ist und deren anderes Ende (18) derart mit einem Zugang (12) zu den Gewebeschichten (2,9) zu verbinden ist, dass das von der Pumpvorrichtung (19) geförderte Fluid zwischen die Gewebeschichten (2,9) zuführbar ist,
mit einer Sensoreinrichtung (21), die dazu eingerichtet ist, Werte, die den Druck im Strömungsweg des Fluids kennzeichnen, zu erfassen und den erfassten Werten entsprechende Signale zu generieren, und
mit einer Steuerungseinrichtung (22), die dazu eingerichtet ist die von der Sensoreinrichtung (21) generierten Signale zu empfangen und aus zu werten, um das einzubringende Volumen des Fluids zu steuern oder zu regeln und unzulässige Veränderungen des Drucks festzustellen.

2. Vorrichtung nach Anspruch 1, **dadurch gekennzeichnet, dass** eine erste Gewebeschicht (2) durch einen Knochen, insbesondere einen Kieferhöhlenknochen, und die von der ersten Schicht zu lösende zweite Gewebeschicht durch eine Membran (9), insbesondere eine Kieferhöhlenschleimhaut, gebildet ist.

3. Vorrichtung nach Anspruch 2, **dadurch gekennzeichnet, dass** sie zur Anwendung bei der Präparation der Kieferhöhlenschleimhaut (9) zur Vorbereitung und/oder Durchführung einer Sinusbodenelevation vorgesehen ist.

4. Vorrichtung nach einem der Ansprüche 1 bis 3, **dadurch gekennzeichnet, dass** das Fluid ein Gas, eine Flüssigkeit oder ein Gas-Flüssigkeitsgemisch ist.

5. Vorrichtung nach Anspruch 4, **dadurch gekennzeichnet, dass** die Pumpvorrichtung (19) eine Dosierpumpe ist, die einen einstellbaren Volumenstrom pumpt.

6. Vorrichtung nach Anspruch 4, **dadurch gekennzeichnet, dass** sie ein Befestigungsmittel (17) umfasst, das dazu dient, die Fluidleitung an einem eine Zugangsöffnung (12) aufweisenden Bereich (5) einer der Gewebeschichten (2) zu befestigen.

7. Vorrichtung nach Anspruch 6, **dadurch gekennzeichnet, dass** das Befestigungsmittel (17) in Form eines an einem Ende (18) der Fluidleitung (15) angeordneten Saugnapfes (35,36) ausgebildet ist.

8. Vorrichtung nach Anspruch 7, **dadurch gekennzeichnet, dass** das Befestigungsmittel (17) zwei unterschiedlich große, konzentrisch zueinander angeordnete Saugnäpfe (18) (35,36) aufweist, die an einem Ende der Fluidleitung angeordnet sind und zwischen denen zur Haftung ein Unterdruck erzeugbar ist.

9. Vorrichtung nach Anspruch 8, **dadurch gekennzeichnet, dass** bei einer Zugangsöffnung (12) von 2 bis 3 mm der erste Saugnapf (35) einen äußeren Rand mit einem Durchmesser von etwa 3 bis 4 mm und der zweite Saugnapf (36) einen äußeren Rand mit einem Durchmesser von etwa 7 bis 16 mm, vorzugsweise 7 bis 9 mm, aufweist.

10. Vorrichtung nach Anspruch 4, **dadurch gekennzeichnet, dass** die Sensoreinrichtung (21) einen Drucksensor (28) umfasst, der den Druck in der Nähe des Pumpenausgangs erfasst.

11. Vorrichtung nach Anspruch 4, **dadurch gekennzeichnet, dass** die Sensoreinrichtung (21) einen Drucksensor (42) umfasst, der den Druckwert direkt an dem Zugang (12) zu den Gewebeschichten (2,9) erfasst.

12. Vorrichtung nach Anspruch 10 oder 11, **dadurch gekennzeichnet, dass** die Steuerungseinrichtung (22) zur Feststellung eines Einrisses der zu lösenden Gewebeschicht (9) den aktuell erfassten Druckwert mit einem Schwellenwert vergleicht.

13. Vorrichtung nach Anspruch 12, **dadurch gekennzeichnet, dass** der Schwellenwert anhand fluidspezifischer und/oder strömungswegspezifischer und/oder objektspezifischer Größen bestimmt wird.

14. Vorrichtung nach Anspruch 4, **dadurch gekennzeichnet, dass** die Pumpvorrichtung eine von einem Motor angetriebene Pumpe ist und die Sensoreinrichtung (22) die für den Druck im Strömungsweg kennzeichnende Leistung des Motors erfasst.

15. Vorrichtung nach Anspruch 1, **dadurch gekennzeichnet, dass** eine Anzeigeeinrichtung (33,34) vorgesehen ist, die beim Feststellen eines Einrisses der zu lösenden Gewebeschicht (9) ein nach außen wahrnehmbares Signal generiert.

16. Vorrichtung nach Anspruch 4, **dadurch gekennzeichnet, dass** die Steuerungseinrichtung (22) die Pumpvorrichtung (19) in Abhängigkeit von Größen steuert oder regelt, zu denen die Art und die Dichte des Fluids und vorzugsweise der Strömungswiderstand im Strömungsweg (15) des Fluids gehören.

17. Vorrichtung nach Anspruch 2 oder 3, **dadurch gekennzeichnet, dass** die Steuerungseinrichtung (22) die Pumpvorrichtung (19) in Abhängigkeit von Größen steuert oder regelt, zu denen die mittlere Länge der Phasen des Ein- und Ausatmens und die dabei bewirkte mittlere Druckdifferenz an dem Zugang (12) zu den Gewebeschichten (2,9) mit berücksichtigt.

18. Vorrichtung nach Anspruch 17, **dadurch gekennzeichnet, dass** die Steuerungseinrichtung (22) die Pumpvorrichtung (19) derart ansteuert oder regelt, dass das Fluid lediglich in den Phasen des Einatmens zugeführt wird.

19. Vorrichtung nach Anspruch 17 oder 18, **dadurch gekennzeichnet, dass** die Zufuhr des Fluids und die Messung der Phasen des Ein- und Ausatmens alternierend zyklisch durchgeführt werden.

## Claims

1. Device (13) for the separation of two tissue layers (2, 9) from one another and/or for the detection of a perforation of a second tissue layer (9) adhering to a first tissue layer (2)
with a pump device (19) which can be connected to a fluid source (26) and is set up for conveying a fluid with volume to be controlled or regulated,
with a fluid line (15), the first end (16) of which can be connected in a flow system with the fluid source (26) and the other end (18) of which is to be connected with an access (12) to the tissue layers (2, 9) so that the fluid conveyed by the pump device (19) can be supplied in between the tissue layers (2, 9),
with a sensor device (21), provided to register data characterizing the pressure within the flow path of the fluid and to generate signals corresponding to the registered data and
with a control device (22), set up to receive and evaluate the signals generated by the sensor device (21) to control or regulate the volume to be fed in and to ascertain improper variations of the pressure.

2. Device according to claim 1, **characterized in that** a first tissue layer (2) is formed by a bone, particularly by a maxillary sinus bone and the second tissue layer, to be separated from the first layer, is formed by a membrane (9), particularly a mandibular sinus membrane.

3. Device according to claim 2, **characterized in that** it is provided for the application at the preparation of the mandibular sinus membrane (9) for preparing and/or performance of a sinus floor elevation.

4. Device according to one of the claims 1 to 3, **characterized in that** the fluid is a gas, a liquid or a mixture of gas and liquid.

5. Device according to claim 4, **characterized in that** the pump device (19) is a dosing pump which pumps an adjustable volume flow rate.

6. Device according to claim 4, **characterized in that** it comprises a fixing means (17) that serves to fix the fluid line at a zone (5) of one of the tissue layers (2), comprising an access opening (12).

7. Device according to claim 6, **characterized in that** the fixing means (17) is formed in the shape of an adhesive bowl (35, 36) disposed at one end (18) of the fluid line (15).

8. Device according to claim 7, **characterized in that** the fixing means (17) comprises two different great adhesive bowls (35, 36), positioned concentrically to one another, which are arranged at one end (18) of the fluid line (18) and between them can be produced a partial vacuum for adhesion.

9. Device according to claim 8, **characterized in that** with an access opening (12) of 2 to 3 mm, the first adhesive bowl (35) comprises an outer edge with a diameter of approximately 3 to 4 mm and the second adhesive bowl (36) comprises an outer edge with a diameter of approximately 7 to 16 mm, preferably 7 to 9 mm.

10. Device according to claim 4, **characterized in that** the sensor device (21) includes a pressure sensor (28) which registers the pressure close to the pump outlet.

11. Device according to claim 4, **characterized in that** the sensor device (21) includes a pressure sensor (42) which senses the pressure value directly at the access (12) to the tissue layers (2, 9).

12. Device according to claim 10 or 11, **characterized in that** the control device (22) compares the current sensed value with a threshold for the determination of an initial tearing of the tissue layer (9) to be separated.

13. Device according to claim 12, **characterized in that** the threshold is determined by means of fluid-specific and/or flowpath-specific and/or object-specific values.

14. Device according to claim 4, **characterized in that** the pump device comprises a pump driven by a motor and the sensor device (22) registers the power of the motor, characteristic for the pressure within the flow path.

15. Device according to claim 1, **characterized in that** a displaying device (33, 34) is provided which generates a signal, outwardly perceptible, when ascertaining an initial tearing of the tissue layer (9) to be separated.

16. Device according to claim 4, **characterized in that** the control device (22) controls or regulates the pump device (19) depending on parameters comprising type and density of the fluid and preferably the flow resistance within the flow path (15) of the fluid.

17. Device according to claim 2 or 3, **characterized in that** the control device (22) controls or regulates the pump device (19) depending on parameters to which the mean length of the phases of intake of air and expulsion of air and the mean pressure difference at the access (12) to the tissue layers (2, 9) is also taken into account.

18. Device according to claim 17, **characterized in that** the control device (22) controls or regulates the pump device (19) in such a way that the fluid is conveyed only during the phases of intake of air.

19. Device according to claim 17 or 18, **characterized in that** the feeding of the fluid and the measurement of the phases of intake of air and expulsion of air are performed in alternating cycles.

## Revendications

1. Dispositif (13) pour la séparation de deux couches de tissu (2, 9) l'une de l'autre et/ou pour la détection d'une perforation dans une seconde couche de tissu (9), adhérente à une première couche de tissu (2)
avec un dispositif de pompage (19), que peut être connecté à une source fluidique (26) et est arrangé pour convoyer un fluide à volume à commander ou à régler,
avec une conduite de fluide (15) dont la première extrémité (16) peut être connectée en ce qui concerne l'écoulement à la source fluidique (26) et dont l'autre extrémité est à connecter à un accès (12) aux couches de tissu (2, 9) de telle façon que le fluide convoyé par le dispositif de pompage (19) peut être amené entre les couches de tissu (2, 9)
avec un dispositif sensoriel (21) installé pour saisir des valeurs, lesquelles indiquent la pression sur trajet d'écoulement du fluide et pour engendrer des signaux correspondants aux valeurs saisies, et
avec un dispositif de commande (22) installé pour recevoir et évaluer les signaux engendrés par le dispositif sensoriel (21) pour commander ou régler le volume du fluide à charger et pour déterminer les modifications inacceptables de pression.

2. Dispositif selon revendication 1, **caractérisé en ce que** une première couche de tissu (2) est formée par un os, particulièrement par un os de sinus mandibulaire et que la deuxième couche de tissu à séparer de la première couche de tissu, est formée par une membrane (9), particulièrement par une membrane d'un sinus mandibulaire.

3. Dispositif selon revendication 2, **caractérisé en ce que** lequel est prévu pour l'application lors de la préparation de la membrane de sinus mandibulaire (9) pour les travaux préparatoires et/ou l'exécution d'une élévation du plancher sinusien.

4. Dispositif selon une des revendications 1 à 3, **caractérisé en ce que** le fluide est un gaz, un liquide ou un mélange gaz-liquide.

5. Dispositif selon revendication 4, **caractérisé en ce que** le dispositif de pompage (19) est une pompe de dosage, laquelle pompe un débit volumétrique ajustable.

6. Dispositif selon revendication 4, **caractérisé en ce que** lequel comprend un moyen de fixation (17) qui sert à fixer la conduite de fluide à une zone (5) d'une des couches de tissu (2) comprenant une ouverture d'accès (12).

7. Dispositif selon revendication 6, **caractérisé en ce que** le moyen de fixation (17) est constitué sous la forme d'une ventouse (35, 36), disposée à une extrémité (18) de la conduite de fluide (15).

8. Dispositif selon revendication 7, **caractérisé en ce que** le moyen de fixation (17) comprend deux ventouses (35, 36), de grandeur différente, disposées concentriquement l'une par rapport à l'autre et lesquelles sont placées à une extrémité de la conduite fluidique (18) et entre lesquelles une dépression peut être générée pour l'adhérence.

9. Dispositif selon revendication 8, **caractérisé en ce que** à une ouverture d'accès (12) de 2 à 3 mm, la première ventouse (35) comprend un bord externe d'un diamètre d'environ 3 à 4 mm et que la deuxième ventouse (36) comprend un bord externe d'un diamètre d'environ 7 à 16 mm, de préférence 7 à 9 mm.

10. Dispositif selon revendication 4, **caractérisé en ce que** le dispositif sensorique (12) comprend un sensor de pression (28), lequel détecte la pression à proximité de la sortie de pompe.

11. Dispositif selon revendication 4, **caractérisé en ce que** le dispositif sensorique (12) comprend un sensor de pression (42), lequel détecte la valeur de pression directement à l'accès (12) auxdites couches de tissu (2, 9).

12. Dispositif selon revendication 10 ou 11, **caractérisé en ce que** le dispositif de commande (22) compare la valeur de pression détectée à l'instant à une valeur seuil pour la constatation d'une déchirure de la couche de tissu (9) à séparer.

13. Dispositif selon revendication 12, **caractérisé en ce que** la valeur seuil est déterminée au moyen des quantités spécifiques au fluide, et/ou au trajet d'écoulement et/ou à l'objet.

14. Dispositif selon revendication 4, **caractérisé en ce que** le dispositif de pompage est une pompe entraînée par un moteur et que le dispositif sensorique (22) détecte la puissance du moteur caractérisant la pression sur le trajet d'écoulement.

15. Dispositif selon revendication 1, **caractérisé en ce qu'**un dispositif indicateur (33, 34) est prévu lequel génère un signal perceptible vers l'extérieur lors de la constatation d'une déchirure de la couche de tissu (9) à séparer.

16. Dispositif selon revendication 4, **caractérisé en ce que** le dispositif de commande (22) actionne ou règle le dispositif de pompage en fonction de quantités dont le genre et la densité du fluide et de préférence la résistance d'écoulement sur le trajet d'écoulement (15) du fluide font partie.

17. Dispositif selon revendication 2 ou 3, **caractérisé en ce que** le dispositif de commande (22) actionne ou règle le dispositif de pompage en fonction de quantités dont le longueur moyenne des phases d'inspiration et d'expiration prend en considération et la pression différentielle moyenne alors produite à l'accès (12) des couches de tissu (2, 9) sont prises en considération.

18. Dispositif selon revendication 17, **caractérisé en ce que** le dispositif de commande (22) actionne ou règle le dispositif de pompage (19) de telle manière que le fluide est amené seulement dans les phases d'inspiration.

19. Dispositif selon revendication 17 ou 18, **caractérisé en ce que** l'amenée du fluide et la mesure des phases d'inspiration et d'expiration sont exécutées de façon alternante cyclique.
